# EUROPEAN PATENT SPECIFICATION

(11) **EP 0 585 104 B1**
(45) Date of publication and mention of the grant of the patent: **19.03.1997**
(21) Application number: 93306693.8
(22) Date of filing: 24.08.1993
(51) Int. Cl.: C07C 69/675, C07C 67/22, C07D 311/00, C07C 253/16, C07D 307/20, C07D 301/02, C07D 307/33, C07C 35/06, C07C 29/147

(54) **A method of preparing a saturated monocyclic hydrocarbon compound and an intermediate therefor**
Verfahren zum Herstellen einer gesättigten monocyclischen Kohlenwasserstoffverbindung und ein Zwischenprodukt für dieses
Méthode de préparation d'un composé hydrocarboné monocyclique saturé et un intermédiaire pour celle-ci

(30) Priority: 25.08.1992 JP 226137/92; 13.08.1993 JP 201605/93
(43) Date of publication of application: 02.03.1994
(73) Proprietor: Japan Tobacco Inc., Shinagawa-ku, Tokyo 140 (JP)
(72) Inventor: Ebata, Takashi, c/o Japan Tobacco Inc., Yokohama-shi, Kanagawa-ken 227 (JP); Koseki, Koshi, c/o Japan Tobacco Inc., Yokohama-shi, Kanagawa-ken 227 (JP); Okano, Koji, c/o Japan Tobacco Inc., Yokohama-shi, Kanagawa-ken 227 (JP); Kawakami, Hiroshi, c/o Japan Tobacco Inc., Yokohama-shi, Kanagawa-ken 227 (JP); Matsumoto, Katsuya, c/o Japan Tobacco Inc., Yokohama-shi, Kanagawa-ken 227 (JP); Matsushita, Hajime, c/o Japan Tobacco Inc., Yokohama-shi, Kanagawa-ken 227 (JP)
(74) Representative: Ruffles, Graham Keith

(56) References cited:
- CHEMICAL ABSTRACTS, vol. 112, no. 9, 26 February 1990, Columbus, Ohio, US; abstract no. 76681z, HIZUKA MICHIYO ET AL 'A stereocontrolled synthesis of trisubstituted cyclohexanes and cyclopentanes.Its application to the synthesis of 11-deoxyprostaglandins' page 750 ;column RIGHT ;

## Description

The present invention relates to a method of preparing a saturated monocyclic hydrocarbon compound and an intermediate therefor which are useful as fragrances of cosmetics and flavors of foods, agricultural chemicals, or synthetic intermediates for pharmaceutical agents.

Methyl dihydroepijasmonate shown below is an fragrance component which provides a jasmine scent and is used widely in cosmetics, foods, or mixed perfumes.

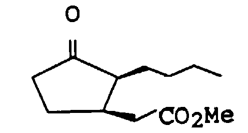

As a fragrance, a compound having two substituting groups in a cis configuration is superior in scent characteristics. Recent studies have reported that a dihydroepijasminic acid derivative having a β-hydroxyl group is concerned in a general plant growth control.

The saturated monocyclic hydrocarbon compound represented by the following formula (1) and methyl dihydroepijasmonate can be widely used as a fragrance component in various foods, cosmetics, and smoking products, as a pharmaceutical agent, as an agricultural chemical, and also as a raw material for synthesizing pharmaceutical agents.

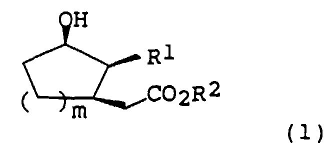

Where, R¹ is an alkyl group having 1 to 10 carbon atoms or an alkenyl group having 2 to 10 carbon atoms, R² is an alkyl group having 1 to 5 carbon atoms, and m is an integer of 1 to 3.

It is known that the saturated monocyclic hydrocarbon compound (1) is generally present in an optically active form and exhibits different scent characteristics depending on the type of an optical isomer. To be more specific, two optical isomers of methyl dihydroepijasmonate are reported to possess different scent characteristics. Hence, it is of great importance to obtain an optical isomer of the saturated monocyclic hydrocarbon compound (1) in optically pure form. However, no reports have been issued on methods to selectively synthesize an optical isomer of the saturated monocyclic hydrocarbon compound (1).

The present invention has been conducted on the basis of the above-mentioned background and provides a method of preparing a saturated monocyclic hydrocarbon compound in which an optically active isomer of that can be selectively obtained and an intermediate therefor.

In one aspect of the present invention, there is provided a method of preparing a saturated monocyclic hydrocarbon compound (1), which comprises the steps of:
(a) obtaining compound (3) by adding an alkyl group or an alkenyl group to an enone site of levoglucosenone (2) as shown in the following reaction: 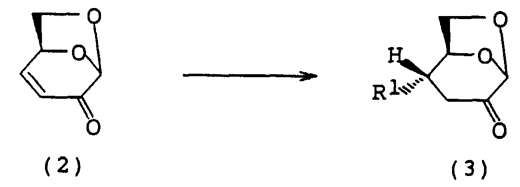 where
   R¹ is an alkyl group having 1 to 10 carbon atoms or an alkenyl group having 2 to 10 carbon atoms, R² is an alkyl group having 1 to 5 carbon atoms, and m is an integer of 1 to 3.
(b) obtaining compound (4) by oxidizing compound (3) obtained in step (a) with a peracid as shown in the following reaction: 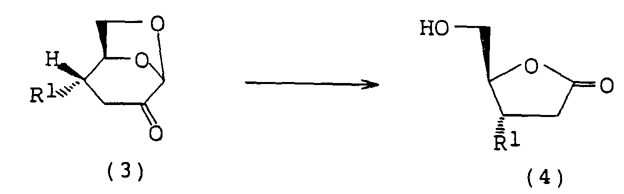 where
   R¹ is the same as defined above.
(c) obtaining compound (5) by converting the hydroxyl group of compound (4) obtained in step (b) into a leaving group by esterification, OX¹ as shown in the following reaction: 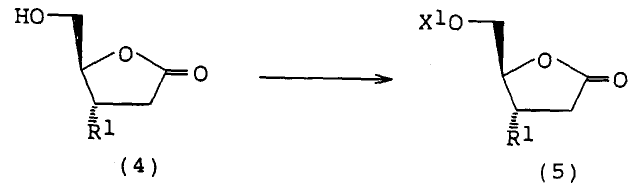 where
   R¹ is the same as defined above, and X¹ is defined as a group capable of being removed in the form of OX¹;
(d) obtaining compound (6) by treating compound (5) obtained in step (c) with a base in the presence of R³OH as shown in the following reaction: 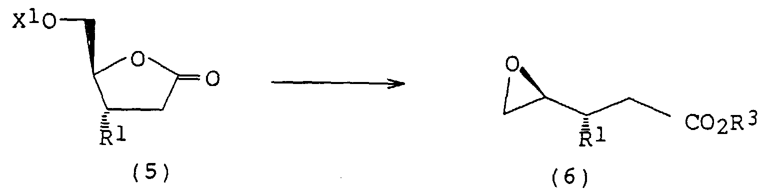 where
   R¹ and X¹ are the same as defined above, and R³ is a hydrogen atom or an alkyl group having 1 to 5 carbon atoms;
(e) obtaining compound (7) by introducing an alkenyl group to compound (6) obtained in step (d) using an alkenyl metal salt as shown in the following reaction: 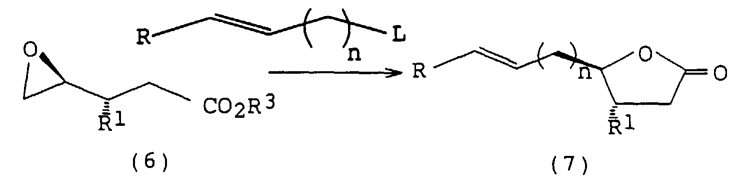 where
   R¹ and R³ are the same as defined above, n is an integer of 1 to 3, R is a hydrogen atom or an alkyl group having 1 to (8-n) carbon atoms, in which n is the same as defined above and L is an alkali metal or MgX (where X is halogen) ;
(f) obtaining compound (8) by oxidatively cleaving the double bond of the alkenyl group of compound (7) obtained in step (e), followed by reducing, as shown in the following reaction: 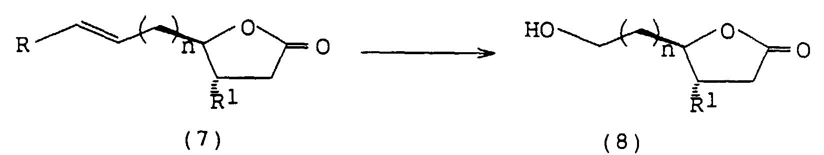 where
   R, R¹ and n are the same as defined above;
(g) obtaining compound (9) by converting the hydroxyl group of compound (8) obtained in step (f) into a leaving group, OX² as shown in the following reaction: 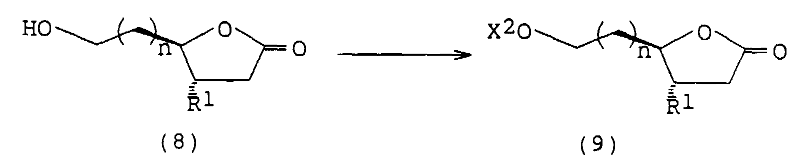 where
   R¹ and n are the same as defined above, and X² is a group capable of being removed in the form of OX²;
(h) obtaining compound (10) by performing a ring-closure reaction of compound (9) obtained in step (g) by treatment with a base as shown in the following reaction: 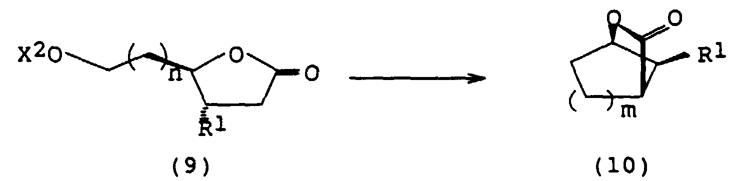 where
   R¹, m, n, and X² are the same as defined above;
(i) obtaining compound (11) by reducing compound (10) obtained in step (h) as shown in the following reaction: 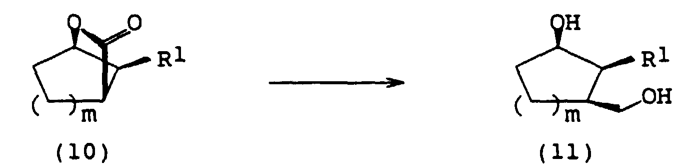 where
   R¹ and m are the same as defined above;
(j) obtaining compound (12) by converting the primary hydroxyl group of compound (11) obtained in step (i) selectively into a leaving group, OX³ as shown in the following reaction: 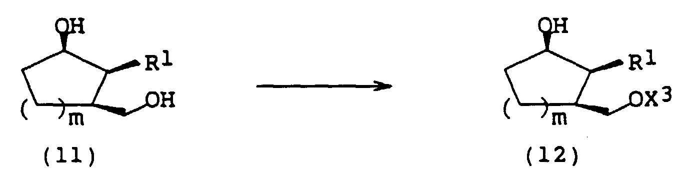 where
   R¹ and m are the same as defined above, and X³ is a group capable of being removed in the form of OX³;
(k) obtaining compound (13) by introducing a protective group, R⁴ to the secondary hydroxyl group of compound (12) obtained in step (j) as shown in the following reaction: 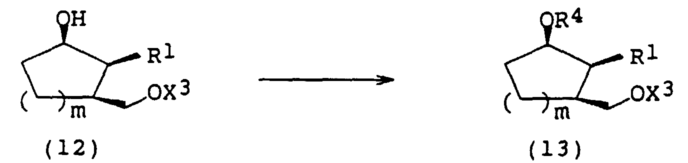 where
   R¹, m, and X³ are the same as defined above, and R⁴ is a protecting group for a hydroxyl group;
(l) obtaining compound represented by chemical formula (14) by introducing a nitrile group to compound represented by chemical formula (13) as shown in the following reaction: 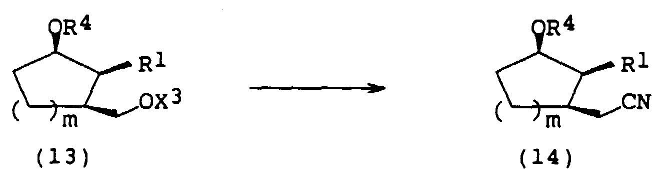 where
   R¹, R⁴, m, and X³ are the same as defined above; and
(m) obtaining a saturated monocyclic hydrocarbon compound (1) by hydrolyzing to convert the nitrile group of compound (14) obtained in step (1) into a carboxyl group, followed by removing the protecting group, R⁴ under an acidic condition, and subsequently performing esterification of the carboxyl group, as shown in the following reaction: 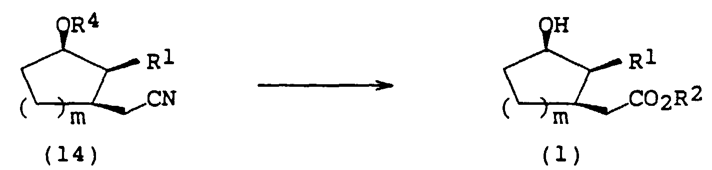 where
   R¹, R², R⁴, and m are the same as defined above.

In another aspect of the present invention, there is provided a method of preparing the following compound (1') comprising the above-mentioned steps (a) to (m), 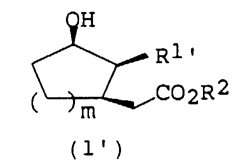 where
R² and m are the same as defined above, and R^{1'} is an alkenyl group having 2 to 10 carbon atoms, wherein an alkenyl group is added to the enone site in place of an alkyl group in step (a);
the product is oxidised as in step (b) resulting in epoxidation of the double bond and formation of a lactone ring, and then said product is further oxidised in order to cleave the carbon-carbon bond of the epoxy ring oxidatively, and then a protection of hydroxyl group and an acetalation are conducted; and

Wittig reaction is performed in the step (m) after the process of a hydrolysis of a nitrile group, deprotection, and esterification.

In a further aspect of the present invention, there is provided compound (10) represented by the following general chemical formula, 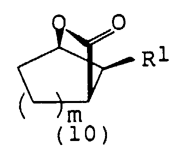 where
R¹ is an alkyl group having 1 to 10 carbon of an alkenyl group having 2 to 10 carbon atoms, and m is an integer of 1 to 3. Racemic compounds of this formula are known from Chem. Pharm. Bull., 1989, 37, 1185.

According to the method of the present invention, there is obtained a saturated monocyclic hydrocarbon compound (1) having high optical purity since synthesis proceeds with keeping the optical purity of a starting levoglucosenone (2). Thus obtained saturated monocyclic hydrocarbon compound (1) is utilized in various scents, pharmaceutical agents, agricultural agents, and the like as it is or by subjecting a further reaction. To be more specific, when the method of the present invention is applied to prepare a saturated monocyclic hydrocarbon compound (1) having a 5 membered ring, a pharmaceutical agent such as prostaglandin is synthesized. When the method is applied to prepare a saturated monocyclic hydrocarbon compound (1) having a 6-membered ring, irone and ionon known as scent, and abscisic acid known as a plant hormone, and the like are synthesized.

Hereinbelow, the method of preparing a saturated monocyclic hydrocarbon compound of the present invention will be described in detail.

A starting material, levoglucosecone of the present invention is widely known as a pyrolysate of cellulose. Levoglucosenone (2) can be readily obtained from galactose with high optical purity in accordance with a process described in Chemistry Letters, 307-310(1990).

Hereinafter, the method of preparing compound (1) which is one aspect of the present invention will be explained.

In step (a), the introduction of an alkyl group R¹ into the enone site of levoglucosenone (2) is performed using an alkylating agent such as alkyl lithium and alkyl magnesium in the presence of a copper salt such as copper (I) iodide, copper (I) bromide, and copper (I) chloride. As alkylating agent, preferably 1.0 to 3.0 mol, more preferably 1.25 mol per mol of levoglucosenone (2) is used. The alkylation reaction is performed in an appropriate solvent under an inert gas generally at a temperature in the range of from -70°C to room temperature for 30 minutes to 20 hours while stirring. The solvent used in this reaction is not restricted, but, for example, an ether-type solvent such as diethyl ether and tetrahydrofuran, toluene, xylene, and the like are used.

In step (b), the reaction to form the 5-membered lactone ring can be performed by Baeyer-Villiger oxidation with an appropriate peracid. For example, 1 to 5 mol, more preferably 1 mol of peracid per mol of compound (3) can be used. As the peracid used in the reaction, there is a peracid such as peracetic acid, performic acid, metachloroperbenzoic acid, peracid of phthalic acid, and the like. The reaction to form the lactone ring is performed by stirring the above-mentioned peracid together with compound (3) in an appropriate solvent at a temperature in the range of from 0 to 50°C for 1 to 80 hours. As the appropriate solvent for use in this reaction, there are an acid such as acetic acid and formic acid, and a halogenated solvent such as methylene chloride and chloroform. The said solvent is not particularly restricted as long as it does not react with peracid and produce by-products which complicate the treatment after the reaction.

In step (c), the reaction to convert a hydroxyl group of compound (4) into a leaving group OX¹ can be performed by the conventional esterification. The OX¹ group functions as a leaving group in the later step (d). Hence, the X¹ group is not restricted as long as it can be removed in the form of OX¹. However, as the X¹ group, a paratoluenesulfonyl group, a methanesulfonyl group, a trifluoromethanesulfonyl group are preferable. The reaction to convert the hydroxyl group of compound (4) into an OX¹ group is performed by stirring the compound (4) together with a halogen compound of X¹ or compound having an X¹ group such as acid anhydride in an appropriate solvent at a temperature in the range of from -10 to 30°C for 3 to 30 hours. In this reaction, 1 to 4 mol, preferably 1.5 mol of the compound having the X¹ group per mol of compound (4) is used. The solvent is not particularly restricted, but, for example, pyridine, triethylamine, and the like can be used.

In step (d), the reaction to convert compound (5) into an epoxy compound can be performed by stirring the compound (5) together with a basic compound in an appropriate solvent generally at a temperature in the range of from 0 to 30°C for 2 to 30 hours. As the basic compound, use is made of, for example, a metal hydroxide such as potassium hydroxide, sodium hydroxide, and lithium hydroxide; carbonate such as potassium carbonate, sodium carbonate; or acetate such as potassium acetate, sodium acetate; and the like. In this reaction, 1 to 3 mol, preferably 1.1 mol of the basic compound per mol of compound (5) can be used. The solvent for use in this reaction is not restricted, however, for example, an alcohol such as methanol, ethanol, isopropyl alcohol, and a mixed solvent of tetrahydrofuran (THF) and the above mentioned alcohol are used. R³ varies depending on the solvent in use. When water or a mixed solvent of water and THF is used, R³ represents a hydrogen atom. When alcohol or a mixed solvent of alcohol and THF is used, R³ represents the alkyl group corresponding to the alcohol in use. However, R³ is not necessary to be restricted since R³ is removed in the subsequent process (e).

The step (e) is a process to obtain compound (7). The compound (7) can be obtained by reacting compound (6) with an alkenyl metal salt such as vinyllithium, vinylmagnesiumhalide represented by a general chemical formula (A): 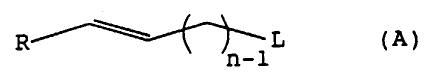 where
R and n are the same as defined above, and L is an alkali metal or MgX (X represents a halogen).

The alkenylation reaction is performed in an appropriate solvent under an inert gas atmosphere at a temperature in the range of from -70°C to room temperature for 1 to 20 hours while stirring. The solvent for use in this reaction is not particularly restricted, however, for example, an ether-type solvent such as ether and tetrahydrofuran, and an organic solvent such as toluene and xylene can be used. The alkenyl group introduced in the 5-position of compound (6) include, for example 1-alkenyl group, 2-alkenyl group, or 3-alkenyl group corresponding to compound (A) in which, n is 1, 2, and 3, respectively. The number of carbon atoms of compound (A) are 2 to 10, preferably 2 to 4. The type of the alkenyl group corresponds to that of the ring formed by a ring-closure reaction in step (h) described later. To be more specific, a 5-membered ring is formed in the case that 1-alkenyl group is used as the alkenyl group; a 6-membered ring in the case of 2-alkenyl group, and a 7-membered ring in the case of 3-alkenyl group.

In step (f), the reaction to obtain an alcohol (8) from compound (7) is performed by oxidatively cleaving a double bond of an alkenyl group, followed by reduction. The oxidative cleavage of the double bond is performed by treating compound (7) with an oxidizing agent such as ozone, ruthenium oxide, osmiumtetraoxide, and sodium periodate. The product obtained from the oxidative cleavage process is then reduced as it is with a reducing agent such as diborane or sodium borohydride. The oxidation and the reduction in this step are performed in an appropriate solvent at a temperature in the range of from -70°C to room temperature for 1 to 30 hours while stirring. The solvent for use in this step is not particularly restricted, but, for example, a halogenated solvent such as carbon tetrachloride, methylene chloride, and the like can be used.

In step (g), the reaction to convert a hydroxyl group to a leaving group OX² is performed by general esterification in substantially the same procedure as in step (c). The X² group can be removed in the form of OX² in the same procedure as in step (c).

The ring-closure reaction in step (h) is performed by an intramolecular alkylation reaction. The intramolecular alkylation reaction of compound (9) is performed by treating compound (9) with an appropriate base. Compound (IX) is obtained from compound (VII) via steps (f) and (g). When compound (IX) is treated with a base, an anion is generated from the 2-position of compound (IX). The resulting anion attacks a carbon atom at the 6-position causing the intramolecular alkylation reaction accompanying the removal of OX². As a result, the ring is formed by ring-closure between a carbon atoms at the 2-and at the 6-position in compound (IX), thereby obtaining compound (X). The type of the formed ring varies depending on the type of the introduced alkenyl group in the process (e). The above-mentioned reaction to obtain compound (X) from compound (VII) via compound (IX) is shown below:

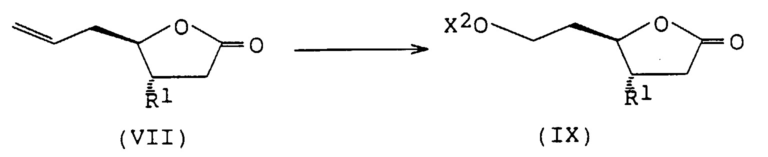

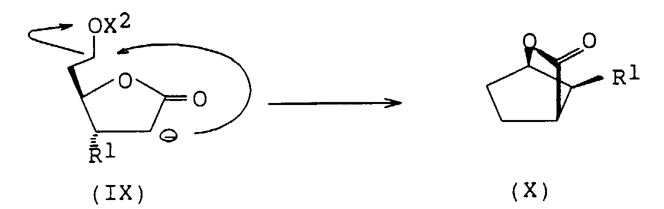

As a base, a strong base such as lithium diisopropylamide, lithium bis(trimethylsilyl)amide is preferably used. The ring-closure reaction is performed in an appropriate solvent under an inert gas atmosphere at a temperature in the range of from -70°C to room temperature for 1 to 30 hours while stirring. The solvent is not particularly restricted, but, for example, ether, tetrahydrofuran, and the like are used.

In step (i), compound (11) is obtained by reductive cleavage of the lactone ring of compound (10). The reduction of compound (10) is performed by treating compound (10) with an appropriate reducing agent. As the reducing agent, for example, lithium aluminum hydride, diisobutylaluminum hydride, and the like can be used. The reduction is performed in an appropriate solvent at a temperature in the range of from -70°C to room temperature for 1 to 30 hours while stirring. The solvent is not particularly restricted, but, for example, an ether-type solvent such as ether and tetrahydrofuran, and an organic solvent such as toluene and xylene.

In the steps (j) to (l), the primary hydroxyl group of two hydroxyl groups of compound (11) is selectively converted into a nitrile group. In step (j), the primary hydroxy group is selectively converted into the OX³ group. The conversion reaction is performed by a general esterification. The X³ group is not particularly restricted since it can be removed in the later step (l). Preferably, a paratoluenesulfonyl group, a methanesulfonyl group, or a trifluoromethanesulfonyl group can be used as X³ group. The conversion reaction of the primary hydroxyl group into the OX³ group is performed by stirring compound (11) together with a compound such as a halogenated compound of X³ or acid anhydride having the X³ group in appropriate solvent at a temperature in the range of from -10 to -30°C for 3 to 30 hours. In this reaction, 1 to 4 mol, preferably 1 mol of compound having the X³ group per mol of compound (11). The solvent is not particularly restricted, but, for example, pyridine, triethylamine, a mixed solvent of a halogenated solvent such as methylene chloride, chloroform and the like, and pyridine, triethylamine, and the like.

In step (k), the protecting group R⁴ introduced to the secondary hydroxyl group is generally used as a protecting group of a hydroxyl group, for example, an acyl type protecting group such as an acetyl group and a benzoyl group; an ether type protecting group such as an ethoxyethyl group and a tetrahydropyranyl group; and a silyl type protecting group such as a t-butyldimethylsilyl group and a triethylsilyl group. When the acyl type protecting group is introduced as the protecting group R⁴, compound (12) can be reacted with, for example, acid anhydride, acyl chloride, or the like in the presence of a basic compound such as pyridine or triethylamine.

When a silyl type protecting group is introduced, compound (12) can be reacted with triorganosilyl chloride or triorganosilyl triflate in the presence of an amine compound such as imidazole and pyridine. The solvent used in this reaction is not particularly restricted, but, an organic solvent such as dimethylformamide, methylene chloride, and the like can be preferably used.

In step (1), the conversion of the primary hydroxy group into a nitrile group is performed by reacting compound (13) with a prussiate such as sodium cyanide and potassium cyanide in an appropriate solvent at a temperature in the range of from room temperature to 180°C for 1 to 30 hours while stirring. The solvent used in this reaction is not particularly restricted, but, for example, dimethylformamide, dimethylsulfoxide, and the like can be used.

In step (m), first, a nitrile group of compound (14) is hydrolyzed to convert it into a carboxyl group. The hydrolysis is performed by using sodium hydroxide or potassium hydroxide as a base in an appropriate solvent at a temperature in the range of from room temperature to 150 °C for 1 to 30 hours while stirring, followed by treating with an appropriate acid. In this reaction, the protective group R⁴ introduced in the secondary hydroxyl group is removed. The solvent used in this reaction is not particularly restricted, but, for example, a mixed solvent consisting of water and the alcohol such as methanol and methoxyethanol can be used. As the acid used in the acid treatment, for example, sulfuric acid, hydrochloric acid, and the like can be used.

The obtained product through the above-mentioned hydrolysis is subjected to reaction with an esterifying agent such as diazomethane in an appropriate solvent to give esterified compound. The solvent used in the esterification reaction is not restricted, but, for example, an organic solvent such as ether and benzene can be used.

Hereinbelow, a method of preparing compound (I') represented by the following general chemical formula, which is the second aspect of the present invention, will be described in detail. 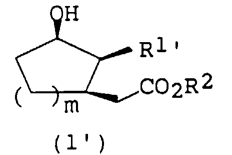 where
R^{1'}, R², and m are the same as defined above.

In step (a), an alkenyl group R^{1'} can be introduced as R¹ into levoglucosenone (2). As a result, compound (1') having an alkenyl group R^{1'} can be obtained as a final product.

The alkenyl group R¹' introduced in the 4-position of levoglucosenone is not particularly restricted, an alkenyl group having 2 to 10 carbon atoms is preferable. The type of an alkenylating agent suitable for the desired alkenyl group can be appropriately chosen.

Hereinbelow, for simplicity's sake, the case in that a 2-alkenyl group is introduced as R¹ will be described. This should not be construed the scope of the present invention.

The introduction of a 2-alkenyl group is performed with an alkenylating agent such as 2-alkenyllithium and 2-alkenylmagnesium halide in place of an alkylating agent employed in step (a) as shown in the following reaction: 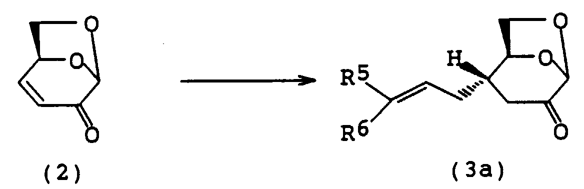 where
R⁵ and R⁶ are a hydrogen atom or an alkyl group.

As shown in the following reaction, step (b) is carried out by using the obtained compound (3a) having 2-alkenyl group. In this step (b), an epoxide (4a) is produced by oxidizing the double bond of the 2-alkenyl group with formation of a lactone ring: 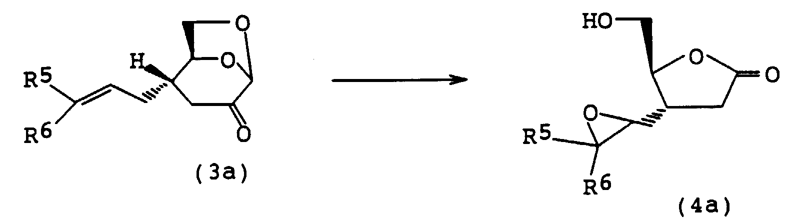 where
R⁵ and R⁶ are a hydrogen atom or an alkyl group.

Then, the epoxide (4a) is reacted with an oxidizing agent such as periodic acid to cleave the carbon-carbon bond of the epoxy ring oxidatively, thereby obtaining compound (4b). Then a protective group R⁷ is introduced in the hydroxyl group at the 5-position of compound (4b), thereby obtaining compound (4c). Further, in order to protect the aldehyde group, the aldehyde group is acetalized, thereby obtaining compound (4d): 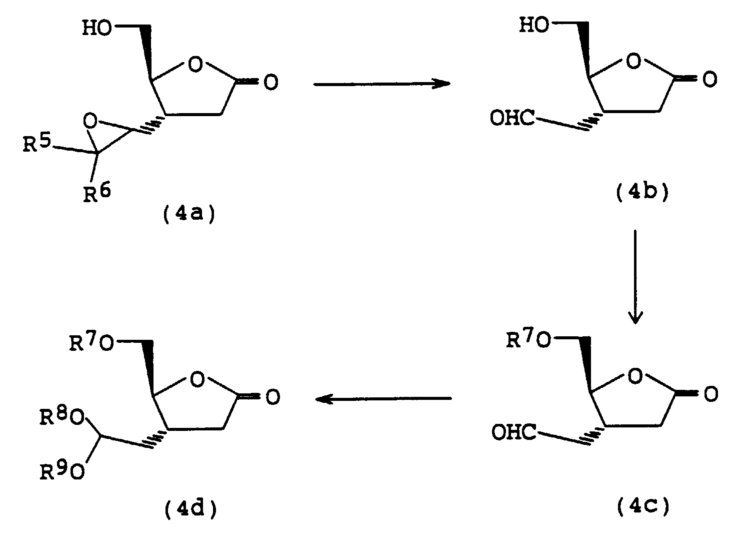 where
R⁵ and R⁶ are the same as defined above; R⁷ is a protecting group for a hydroxyl group; R⁸ and R⁹ are an alkyl group which can be the same; and R⁸ and R⁹ can be bound to each other to form a ring.

To remove the protecting group R⁷ of the hydroxyl group of the obtained compound (4d), the above-mentioned steps (c) to (l) are carried out.

Then, step (m) is performed using the obtained compound (14a) in step (l). Compound (14a) is subjected to hydrolysis of the nitrile group, esterification, and removal of the protecting group R⁴ with convertion of the acetal group into an aldehyde group, thereby obtaining compound (15a). The reaction is shown below: 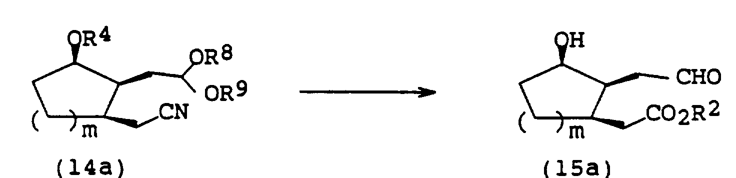 where
R², R⁴, R⁸ and R⁹ are the same as defined above.

The obtained compound (15a) is subjected to Wittig reaction, thereby obtaining compound (1'a) having 2-alkenyl group as R¹ of compound (1). The reaction is shown below: 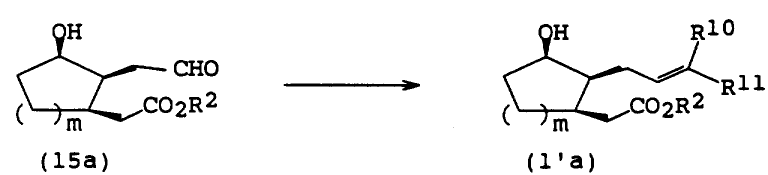 where
R² is the same as defined above, and R¹⁰ and R¹¹ are a hydrogen atom or an alkyl group.

The compound (15a) can be subjected to Wittig reaction either directly or after converting the aldehyde group to a carboxyl group.

From the foregoing, compound (1') having an alkenyl group R^{1'} as R¹ of compound (1) is obtained. 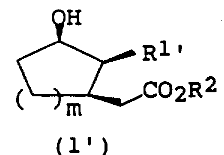 where
R^{1'}, R² and m are the same as defined above.

### Example

Hereinafter, examples, in which methyl dihydroepijasmonate was produced by applying a method of preparing a saturated monocyclic hydrocarbon compound will be described in detail.

### [A] Synthesis of 1,6-anhydro-3,4-dideoxy-4-C-pentyl-β-D-erythro-hexopyranose-2-ulose [compound (3)]

A solution of levoglucosenone [3.51 g (925 mmol)] in anhydrous ether (10 ml) was added dropwise at -60°C to a lithium dimethylcuprate solution which has been prepared by using copper iodide [5.72g (30 mmol)] and pentyllithium [75 ml (0.8N, 60 mmol)] according to a conventional method. Thereafter, the solution was stirred at -60 °C for 30 minutes, the reaction temperature was raised to 0°C. Then, the reaction mixture was poured into a saturated aqueous ammonium chloride solution. After the reaction mixture was stirred at room temperature for 30 minutes, an insoluble material was filtered off. From the obtained filtrate, the organic layer was separated and the water layer was subjected to extraction with methylene chloride. The obtained extract was combined with the previously separated organic layer, followed by washing with a saturated aqueous sodium chloride solution, and dried with anhydrous magnesium sulfate. After filtration, the filtrate was concentrated and the obtained residue was purified by means of silica gel column chromatography (n-hexane : ether = 10 : 1 - 2 : 1). The purified product is further distilled under reduced pressure. As a result, compound (3) is obtained in a yield of 4.01 g (81.0%).

The obtained product has the following physical properties:
Boiling point: 113 - 115°C/10 mmHg
¹HNMR (CDCℓ₃): δ
0.89 (3H, t, J = 7.0 Hz),
1.20 - 1.72 (8H, m)
2.01 - 2.22 (2H, m)
2.80 (1H, dd, J = 7.8, 16.3 Hz),
3.92 - 4.06 (2H, m),
4.50 - 4.59 (1H, m),
5.06 (1H, s).

### [B] Synthesis of (3S,4S)-5-hydroxy-3-pentylpentane-4-olide [compound (4)]

The compound (3) obtained in step [A] [7.51 g (37.9 mmol)] was dissolved in acetic acid (37 ml), and further 40% peracetic acid (6.7 ml) was added dropwise thereto while stirring. During this reaction, the temperature was maintained at a temperature in the range of 20 to 30°C. After the reaction mixture was stirred at room temperature overnight, dimethylsulfide [2.40 g (38.7 mmol)] was added dropwise to the reaction mixture under cooling, followed by stirring at room temperature for 30 minutes. After the resultant solution was concentrated, the obtained residue was dissolved in methanol (30 ml), further 10 drops of concentrated hydrochloric acid was added thereto. The solution was stirred for 6 hours while warming the temperature to 50°C. The solution was concentrated and the obtained residue was purified by means of silica gel column chromatography (n-hexane : ethyl acetate = 1 : 1 - 1 : 3). As a result, compound (4) was obtained in a yield of 6.38 g (90.5%).

The obtained product has the following physical properties:
¹HNMR (CDCℓ₃): δ
0.90 (3H, t, J = 6.9 Hz),
1.10 - 1.62 (9H, m)
2.22 (1H, dd, J = 8.7, 17.3 Hz)
2.35 - 2.52 (1H, m),
2.75 (1H, dd, J = 8.6, 17.3 Hz)
3.61 - 3.74 (1H, m),
3.85 - 3.96 (1H, m),
4.19 - 4.29 (1H, m).

### [C] Synthesis of (3S,4S)-3-pentyl-5-tosyloxypentane-4-olide [compound (5)]

The compound (4) obtained in step [B] [1.51 g (8.12 mmol)] was dissolved in anhydrous pyridine (10 ml), and further tosyl chloride [2.32 g (12.2 mmol)] was added thereto under ice-cooling. After being stirred at room temperature for 3 hours, the reaction mixture was poured into a cold diluted hydrochloric acid solution and subjected to extraction with ether. The obtained ether extract was successively washed with water, a saturated aqueous copper sulfate solution, water and a saturated aqueous sodium chloride solution, and dried with anhydrous magnesium sulfate. The solution was filtered and the filtrate was concentrated. The obtained residue was purified by means of silica gel column chromatography (n-hexane : ethyl acetate = 10 : 1 - 5 : 1). As a result, compound (5) was obtained in a yield of 2.39 g (86.6%).

The obtained product has the following physical properties:
¹HNMR (CDCℓ₃): δ
0.90 (3H, t, J = 6.9 Hz),
1.20 - 1.61 (8H, m),
2.21 (1H, dd, J = 8.1, 17.4 Hz),
2.30 - 2.53 (4H, m),
2.73 (1H, dd, J = 8.6, 17.4 Hz),
4.10 - 4.34 (3H, m),
7.39 (2H, d, J = 8.5 Hz),
7.80 (2H, d, J = 8.5 Hz).

### [D] Synthesis of isopropyl (3S,4S)-4,5-epoxy-3-pentylpentanoate [compound (6)]

The compound (5) [3.00 g (8.82 mmol)] obtained in step [C] was dissolved in isopropyl alcohol (50 ml), and further potassium carbonate [1.34 g (9.71 mmol) was added thereto. After the reaction mixture was stirred at 50 °C overnight, an insoluble material was filtered off. The filtrate was concentrated and the obtained residue was purified by means of column chromatography (n-hexane : ether = 10 : 1 - 5 : 1). As a result, an epoxide (6) is obtained in a yield of 1.53 g (76.1%).

The obtained product has the following physical properties:
¹HNMR (CDCℓ₃): δ
0.89 (3H, t, J = 6.9 Hz),
1.15 - 1.50 (15H, m),
2.32 (1H, dd, J = 8.3, 15.3 Hz),
2.49 (1H, dd, J = 7.8, 15.3 Hz),
2.52 - 2.60 (1H, m),
2.75 - 2.86 (2H, m),
4.96 - 5.10 (1H, m).

### [E] Synthesis of (3S,4R)-3-pentyl-6-hepten-4-olide [compound (7)]

Copper (I) iodide [129 mg (0.68 mmol)] was suspended in anhydrous tetrahydrofuran (5 ml), and further a solution of epoxide (6)[770 mg (3.38 mmol) obtained in step [D] in anhydrous ether (25 ml) was added thereto. To the mixture, was added dropwise a solution of vinylmagnesium bromide in tetrahydrofuran [3.71 ml (1.0N, 3.71 mmol)] while cooling at -20°C under an argon gas atmosphere. After being stirred at -20°C for one hour, the reaction mixture was poured into a saturated aqueous ammonium chloride solution and further stirred for 30 minutes. After an insoluble material was filtered off, the obtained filtrate was subjected to extraction with ether and then the ether extract was washed with a saturated aqueous sodium chloride solution. Thereafter, the ether extract was dried with anhydrous magnesium sulfate and filtered. The filtrate was concentrated and the obtained residue was dissolved in methanol (5 ml), and further, 10% aqueous sodium hydroxide solution (5 ml) was added thereto, followed by stirring at room temperature for 3 hours. Methanol was removed by distillation under reduced pressure and the obtained residue was subjected to extraction twice with ether. After the obtained water layer was acidified with diluted hydrochloric acid, the mixture was stirred for one hour. Then, the water layer was subjected to extraction with ether. The obtained ether extract was dried with anhydrous magnesium sulfate and concentrated to obtain a crude product. The crude product was purified by means of silica gel column chromatography (n-hexane : ether = 10 : 1 - 5 : 1). As a result, compound (7) was obtained in a yield of 469 mg (70.8%).

The obtained product has the following physical properties:
¹HNMR (CDCℓ₃): δ
0.89 (3H, t, J = 6.7 Hz),
1.17 - 1.65 (8H, m),
2.12 - 2.28 (2H, m),
2.33 - 2.54 (2H, m),
2.68 (1H, dd, J = 11.5, 20.5 Hz),
4.17 (1H, dd, J = 6.5, 11.5 Hz),
5.12 - 5.22 (2H, m),
5.74 - 5.90 (1H, m).

### [F] Synthesis of (3S,4R)-6-hydroxy-3-pentyl-4-hexanolide [compound (8)]

The compound (7)[327 mg (1.67 mmol)] obtained in step [E] was dissolved in anhydrous methylene chloride (40 ml), and further ozone was bubbled until the solution was saturated while cooling at -78°C. After the temperature of the reaction mixture was raised to room temperature, an excess ozone was removed by bubbling an argon gas. Then, borane-dimethyl sulfide complex [0.58 ml(10M. 5.84 mmol)] was added to the reaction mixture, followed by stirring at room temperature for 20 hours. To the resultant solution, 1N diluted hydrochloric acid (1 ml) was added and the mixture was stirred for one hour. Sodium hydrogen carbonate powder was added until the solution was changed to a basic condiction. Then anhydrous magnesium sulfate was added to dry the solution and subjected to filtration. The filtrate was concentrated and the obtained residue was purified by means of column chromatography (n-hexane : ethyl acetate = 10 : 1 - 3 : 1). As a result, compound (8) was obtained in a yield of 272 mg (81.9%).

The obtained product has the following physical properties:
¹HNMR (CDCℓ₃): δ
0.90 (3H, t, J = 6.8 Hz),
1.21 - 1.66 (7H, m),
1.76 - 2.30 (6H, m),
2.61 - 2.79 (1H, m),
3.74 - 3.89 (2H, m),
4.25 - 4.36 (1H, m).

### [G] Synthesis of (3S,4R)-3-pentyl-6-tosyloxyhexan-4-olide [compound (9)]

The compound [8] [200 mg (1.01 mmol)] obtained in step [F] was dissolved in anhydrous pyridine (5 ml), and further tosyl chloride [287 mg (1.51 mmol] was added thereto under ice-cooling. After being stirred at room temperature overnight, the reaction mixture was poured into a diluted aqueous hydrochloric acid solution and subjected to extraction with ether. The obtained extract was successively washed with water, a saturated aqueous copper sulfate solution water and a saturated aqueous sodium chloride solution, and dried with anhydrous magnesium sulfate. The organic layer was concentrated and the obtained residue was purified by means of silica gel column chromatography (n-hexane : ethyl acetate = 10 : 1 - 5 : 1). As a result, compound (9) was obtained in the yield of 292 mg (82.3%).

The obtained product has the following physical properties:
¹HNMR (CDCℓ₃): δ
0.90 (3H, t, J = 6.8 Hz),
1.18 - 1.59 (8H, m),
1.80 - 1.93 (1H, m),
2.03 - 2.27 (3H, m),
2.56 (3H, s),
2.65 (1H, dd, J = 8.0, 16.9 Hz),
4.06 - 4.26 (3H, m),
7.38 (2H, d, J = 8.5 Hz),
7.79 (2H, d, J = 8.5 Hz).

### [H] Synthesis of (1R,4S,7S)-7-pentyl-2-oxabicyclo[2.2.1]heptan-3-one [compound (10)]

The compound (9) [197 mg (0.56 mmol)] obtained in step [G] was dissolved in anhydrous tetrahydrofuran (5 ml), and further a solution of lithium bis(trimethylsilyl)amide in tetrahydrofuran [0.56 ml, (1N, 0.56 mmol)] was added dropwise thereto under an argon gas atmosphere while cooling at -78°C. After the reaction temperature was gradually raised to room temperature in 3 hours, the reaction mixture was poured into a saturated aqueous ammonium chloride solution and subjected to extraction with ether. After the obtained extract was washed with a saturated aqueous sodium chloride solution and dried with anhydrous magnesium sulfate, the resultant solution was filtered and the filtrate was concentrated. The obtained residue was purified by means of silica gel column chromatography (n-hexane : ether = 10 : 1). As a result, compound (10) was obtained in a yield of 87.3 mg (85.6%).

The obtained product has the following physical properties:
¹HNMR (CDCℓ₃) : δ
0.90 (3H, t, J = 6.8 Hz),
1.19 - 1.60 (9H, m),
1.62 - 1.80 (1H, m),
1.81 - 2.06 (3H, m),
2.68 - 2.75 (1H, m),
4.60 - 4.68 (1H, m).

### [I] Synthesis of (1R,2S,3R)-3-hydroxymethyl-2-pentyl-1-cyclopentanol [compound (11)]

The compound (10) (21 mg, 0.21 mmol) obtained in step [H] was dissolved in anhydrous ether (1 ml), and further lithium aluminum hydride (6.6 mg, 0.17 mmol) was added thereto while stirring under ice-cooling. After the reaction mixture was stirred for one hour, first water (0.1 ml), and then 2N sodium hydroxide (0.2 ml), and subsequently water (0.1 ml) were added to the reaction mixture, and stirred for a while, and then filtered to remove an insoluble material. The filtrate was concentrated, and the obtained residue was purified by means of silica gel column chromatography (n-hexane : ethyl acetate = 10 : 1 - 1 : 1). As a result, a diol compound (11) was obtained in a yield of 18 mg (83.7%).

The obtained product has the following physical properties:
¹HNMR (CDCℓ₃) : δ
0.89 (3H, t, J = 6.6 Hz),
1.21 - 1.60 (6H, m),
1.67 - 1.98 (5H, m),
2.16 - 2.29 (1H, m),
3.42 - 3.73 (4H, m),
4.05 - 4.14 (1H, m).

### [J] Synthesis of (1R,2S,3R)-3-tosyloxymethyl-2-pentyl-1-cyclopentanol [compound (12)]

The diol compound (11) [23 mg, (0.12 mmol)] obtained in step [H] was dissolved in anhydrous methylene chloride (1 ml), and further a solution of tosyl chloride [24 mg, (0.12 mmol)] and a solution of pyridine [29 mg, (0.37 mmol)] in anhydrous methylene chloride (1 ml) was slowly added dropwise thereto while stirring under ice-cooling. After the reaction mixture was stirred at room temperature overnight, a saturated aqueous sodium chloride solution (1 ml) was added to the reaction mixture. The resultant solution was subjected to extraction with ether and the obtained ether extract was washed first with a saturated aqueous copper sulfate solution, subsequently with a saturated aqueous sodium chloride solution, and dried with anhydrous magnesium sulfate. Then, the extract was filtered and the filtrate was concentrated. The obtained residue was purified by means of silica gel column chromatography (n-hexane : ethyl acetate = 10 : 1 - 1 : 1). As a result, as a monotosylate, compound (12) was obtained in a yield of 42.8 mg (81.7%).

The obtained product has the following physical properties:
¹HNMR (CDCℓ₃): δ
0.87 (3H, t, J = 6.8 Hz),
1.18 - 1.40 (7H, m),
1.52 - 1.85 (7H, m),
2.24 - 2.38 (1H, m),
2.45 (3H, s),
3.99 - 4.17 (3H, m),
7.34 (2H, m),
7.79 (2H, m).

### [K] Synthesis of (1R,2S,3R)-2-pentyl-1-tetrahydropyranyloxy-3-tosyloxymethylcyclopentane[compound (13)]

The monotosylate (12) [23 mg (0.054 mmol)] obtained in step [J] was dissolved in anhydrous methylene chloride (2 ml), and further dihydropyrane [14 mg (0.16 mmol)] and pyridinium paratoluenesulfonate (3 mg) were added thereto and stirred at room temperature overnight. To the reaction mixture, first ether (3 ml), and subsequently a 50% saturated aqueous sodium chloride solution were added, and then the organic layer was separated. After being dried with anhydrous magnesium sulfate, the organic layer was filtered and the filtrate was concentrated. The obtained residue was purified by means of preparative thin-layer chromatography (n-hexane : ether = 1 : 1). As a result, tosylate (13) was obtained in a yield of 25.3 mg (91.7%).

The obtained product has the following physical properties:
¹HNMR (CDCℓ₃): δ
0.87 (3H, t, J = 6.7 Hz),
1.19 - 2.00 (19H, m),
2.22 - 2.39 (1H, m),
2.44 (3H, m),
3.39 - 3.50 (1H, m),
3.62 - 3.80 (1H, m),
3.87 - 4.19 (3H, m),
4.51 - 4.59 (1H, m),
7.31 - 7.37 (2H, m),
7.75 - 7.84 (2H, m).

### [L] Synthesis of (1R,2S,3R)-3-cyanomethyl-2-pentyl-1-tetrahydropyranyloxycyclopentane [compound (14)]

Tosylate (13) [20 mg (0.04 mmol)] obtained in step [K] was dissolved in anhydrous dimethylformamide (1 ml), and further sodium cyanate [5.8 mg (0.12 mmol)] and tetrabutylammonium bromide [2.9 mg (0.009 mmol)] were added thereto while stirring, followed by stirring at 100°C overnight. After being cooled, the reaction mixture was poured into water and subjected to extraction with ether. The obtained ether extract was washed with a saturated aqueous sodium hydrogen carbonate solution and then dried with anhydrous magnesium sulfate. Subsequently, the extract was filtered and the filtrate was concentrated. The obtained residue was purified by means of silica gel column chromatography (n-hexane : ether = 20 : 1 - 5 : 1). As a result, the nitrile compound (14) was obtained in a yield of 8.3 mg (58.0%).

The obtained product has the following physical properties:
¹HNMR (CDCℓ₃): δ
0.89 (3H, t, J = 6.9 Hz),
1.20 - 2.05 (18H, m),
2.28 - 2.55 (3H, m),
3.45 - 3.56 (1H, m),
3.75 - 3.90 (1H, m),
3.97 - 4.03 (0.7H, m),
4.14 - 4.19 (0.3H, m),
4.52 - 4.59 (0.7H, m),
4.63 - 4.68 (0.3H, m).

### [M] Synthesis of (1R,2S,3R)-1-hydroxy-3-methoxycarbonylmethyl-2-pentylcyclopentane [compound (1)]

The nitrile compound (14) [28 mg (0.077 mmol)] obtained in step [L] was dissolved in methanol (1 ml), and further a solution of a sodium hydroxide [28 mg (0.7 mmol)] in water (1 ml) was added thereto. After stirred at 60°C for 6 hours, the reaction mixture was poured into a diluted aqueous sulfuric acid solution. After stirring for one hour, the reaction mixture was subjected to extraction with methylene chloride. The obtained extract was washed with water, subsequently with a saturated aqueous sodium chloride solution, and then dried with anhydrous magnesium sulfate. The extract was filtered and the obtained filtrate was concentrated. The obtained residue was dissolved in ether (1 ml), and further a solution of diazomethane in ether (0.2 ml) was added thereto and stirred for one hour. The mixture was then concentrated and the obtained residue was purified by means of silica gel column chromatography (n-hexane : ethyl acetate = 10 : 1 - 5 : 1). As a result compound (1) as a hydroxyl ester was obtained in a yield of 15.9 mg (90.3%).

The obtained product has the following physical properties:
¹HNMR (CDCℓ₃): δ
0.90 (3H, t, J = 7.0 Hz),
1.10 - 2.45 (17H, m),
3.66 (3H, s),
4.13- 4.27 (1H, m).

### [N] Synthesis of (2S,3R)-3-methoxycarbonylmethyl-2-pentyl-1-cyclopentanone (methyl dihydroepijasmonate)

Hydroxyl ester (1)[20 mg (0.088 mmol)] obtained in step [M] was dissolved in anhydrous methylene chloride, and further pyridinium dichromate [49 mg (0.13 mmol) was added thereto and stirred at room temperature for 3 hours. To the resultant solution, ether was added and an insoluble material was removed by means of Florisil column. The fraction containing the product was concentrated and the obtained residue was purified by means of silica gel column chromatography (n-hexane : ether = 10 : 1 - 5 : 1). As a result, methyl dihydroepijasmonate in a yield of 12.4 mg (62.6%) was obtained.

The obtained product has the following physical properties:
¹HNMR (CDCℓ₃): δ
0.88 (3H, t, J = 6.9 Hz),
1.16 - 1.60 (9H, m),
1.73 - 1.80 (2H, m),
2.07 - 2.17 (1H, m),
2.19 - 2.26 (1H, m),
2.27 - 2.38 (2H, m),
2.58 - 2.69 (1H, m),
3.71 (3H, s).

Methyl dihydroepijasmonate is useful as a fragrance component of cosmetics.

As described above, according to the method of the present invention, an optically active saturated monocyclic hydrocarbon compound maintaining the optical purity of as the starting material can be obtained by using as a starting material levoglucosenone with high optical purity which is easily obtained.

## Claims

1. A method of preparing a saturated monocyclic hydrocarbon compound represented by the following chemical formula (1): 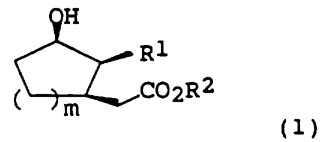 where
R¹ is an alkyl group having 1 to 10 carbon atoms or an alkenyl group having 2 to 10 carbon atoms, R² is an alkyl group having 1 to 5 carbon atoms, and m is an integer of 1 to 3,
said method comprising the steps of:
(a) obtaining compound (3) by adding an alkyl group or an alkenyl group to an enone site of levoglucosenone (2) as shown in the following reaction: 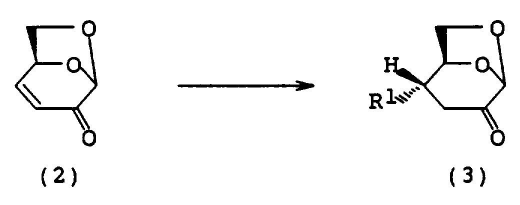 where
R¹ is the same as defined above;
(b) obtaining compound (4) by oxidizing compound (3) obtained in step (a) with a peracid as shown in the following reaction: 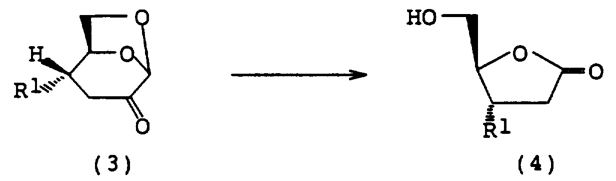 where
R¹ is the same as defined above;
(c) obtaining compound (5) by converting the hydroxyl group of compound (4) obtained in step (b) into a leaving group by esterification, OX¹ as shown in the following reaction: 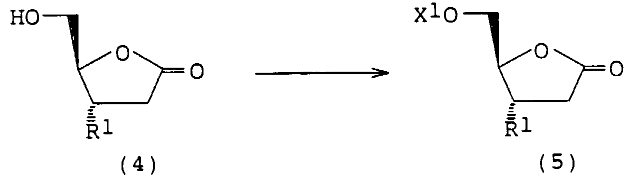 where
R¹ is the same as defined above, and X¹ is defined as a group capable of being removed in the form of OX¹;
(d) obtaining compound (6) by treating compound (5) obtained in step (c) with a base in the presence of R³OH as shown in the following reaction: 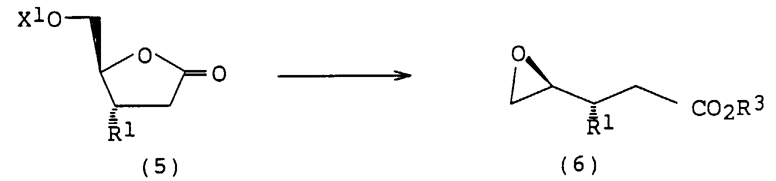 where
R¹ and X¹ are the same as defined above, and R³ is a hydrogen atom or an alkyl group having 1 to 5 carbon atoms;
(e) obtaining compound (7) by introducing an alkenyl group to compound (6) obtained in step (d) using an alkenyl metal salt as shown in the following reaction: 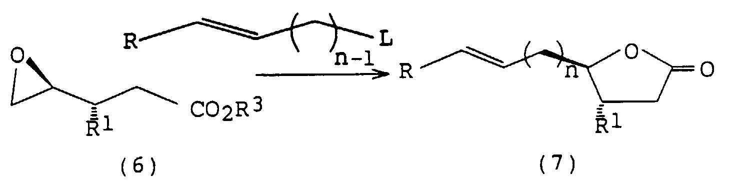 where
R¹ and R³ are the same as defined above, n is an integer of 1 to 3, R is a hydrogen atom or an alkyl group having 1 to (8-n) carbon atoms, in which n is the same as defined above and L is an alkali metal or MgX (where X is halogen);
(f) obtaining Compound (8) by oxidatively cleaving the double bond of the alkenyl group of compound (7), obtained in step (e), followed by reducing, as shown in the following reaction: 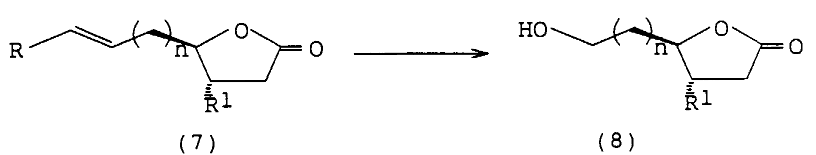 where
R, R1 and n are the same as defined above;
(g) obtaining compound (9) by converting the hydroxyl group of compound (8) obtained in step (f) into a leaving group, OX² as shown in the following reaction: 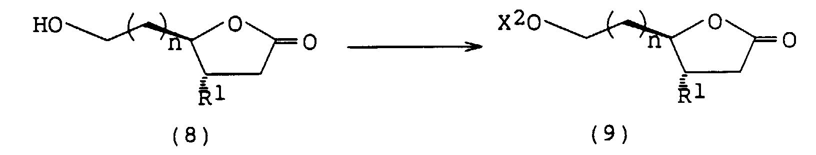 where
R¹ and n are the same as defined above, and X² is a group capable of being removed in the form of OX²;_{,}
(h) obtaining compound (10) by performing a ring-closure reaction of compound (9) obtained in step (g) by treatment with a base as shown in the following reaction: 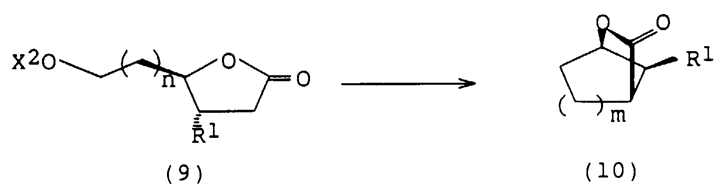 where
R¹, m, n, and X² are the same as defined above;
(i) obtaining compound (11) by reducing compound (10) obtained in step (h) as shown in the following reaction: 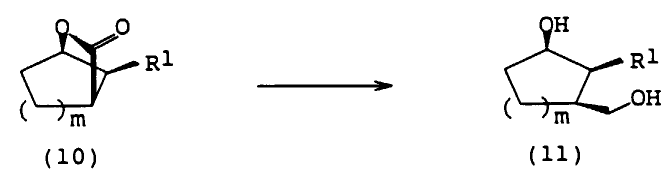 where
R¹ and m are the same as defined above;
(j) obtaining compound (12) by converting the primary hydroxyl group of compound (11) obtained in step (i) selectively into a leaving group, OX³ as shown in the following reaction: 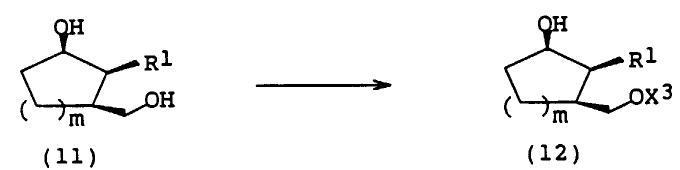 where
R¹ and m are the same as defined above, and X³ is a group capable of being removed in the form of OX³;
(k) obtaining compound (13) by introducing a protective group, R⁴ to the secondary hydroxyl group of compound (12) obtained in step (j) as shown in the following reaction: 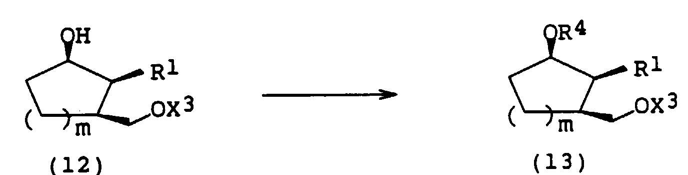 where
R¹, m, and X³ are the same as defined above, and R⁴ is a protecting group of a hydroxyl group;
(l) obtaining compound represented by chemical formula (14) by introducing a nitrile group to compound represented by chemical formula (13) as shown in the following reaction: 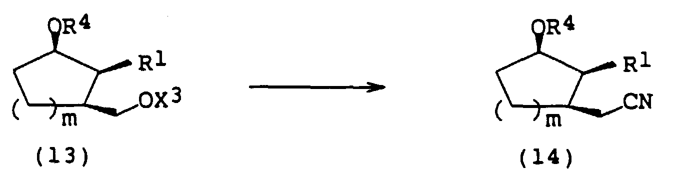 where
R¹, R⁴, m, and X³ are the same as defined above; and
(m) obtaining a saturated monocyclic hydrocarbon compound (1) by hydrolyzing to convert the nitrile group of compound (14) obtained in step (1) into a carboxyl group, followed by removing the protecting group, R⁴ under an acidic condition, and subsequently performing esterification of the carboxyl group, as shown in the following reaction: 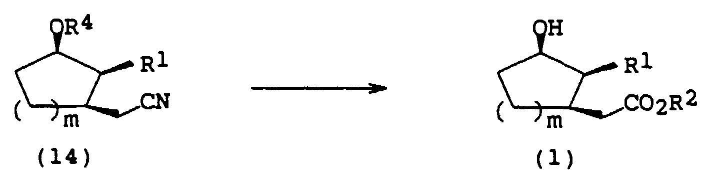 where
R¹, R², R⁴, and m are the same as defined above.

2. A method of preparing a saturated monocyclic hydrocarbon compound comprising said steps (a) to (m) according to claim 1, characterized in that said compound (1) is represented by the following general chemical formula (1'): 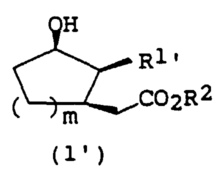 where
R² and m are the same as defined above, and R^{1'} is an alkenyl group having 2 to 10 carbon atoms, and wherein,
an alkenyl group is added to the enone site in place of an alkyl group used in step (a); the product is oxidized as in step (b) resulting in epoxidation of the double bond and formation of a lactone ring, and then said product is further oxidized in order to cleave the carbon-carbon bond of the epoxy ring oxidatively, and then a protection of the hydroxyl group and an acetalation are conducted; and Wittig reaction is performed in the step (m) after a process of a hydrolysis of nitrile group, deprotection, and esterification.

3. The method according to claim 1, characterized in that said step (a) is performed by using a metal alkylate in the presence of a copper (I) salt.

4. The method according to claim 2, characterized in that said step (a) is performed by using a metal alkenylate in the presence of a copper (I) salt.

5. The method according to either claim 1 or claim 2, characterized in that said step (e) is performed by using a metal alkenylate in the presence of a copper (I) salt.

6. The method according to claim 3, characterized in that said metal alkylate is alkyllithium or alkylmagnesium halide having 1 to 10 carbon atoms.

7. The method according to claim 4, characterized in that said metal alkenylate is alkenyllithium or alkenylmagnesium halide having 2 to 10 carbon atoms.

8. The method according to claim 5, characterized in that said metal alkenylate is alkenyllithium or alkenylmagnesium halide having 2 to 10 carbon atoms.

9. The method according to either claim 3 or claim 4, characterized in that said copper (I) salt is copper (I) iodide, copper (I) bromide, and copper (I) chloride.

10. The method according to either claim 1 or claim 2, characterized in that the groups X¹, X², and X³ in said steps (c), (g) and (j) are introduced by an esterification reaction.

11. The method according to claim 1, characterized in that said compound (1) is represented by the following chemical formula:

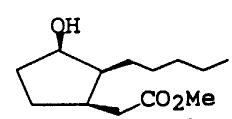

12. The compound represented by the following general chemical formula (10): 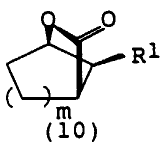 where
R¹ is an alkyl group having 1 to 10 carbon atoms or an alkenyl group having 2 to 10 carbon atoms, and m is an integer of 1 to 3.

## Patentansprüche

1. Verfahren zur Herstellung einer gesättigten monozyklischen Kohlenwasserstoffverbindung der nachfolgenden chemischen Formel (1): 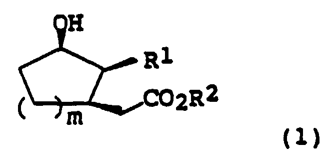 wobei R¹ eine Alkylgruppe mit 1 bis 10 Kohlenstoffatomen oder eine Alkenylgruppe mit 2 bis 10 Kohlenstoffatomen ist, R² eine Alkylgruppe mit 1 bis 5 Kohlenstoffatomen ist und m ein Ganzzahliges von 1 bis 3 ist,
wobei das Verfahren die nachfolgenden Schritte umfasst:
(a) Erhalten der Verbindung (3) durch anfügen einer Alkylgruppe oder einer Alkenylgruppe an die Enonstelle des Lävoglukosenons (2) gemäß der nachfolgenden Reaktion: 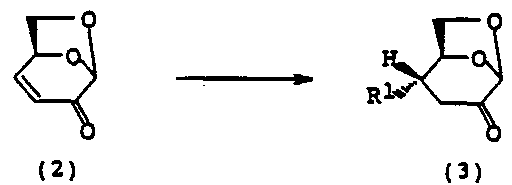 wobei R¹ die gleichen Bedeutungen wie oben definiert aufweist;
(b) Erhalten der Verbindung (4) durch Oxidieren der im Schritt (a) erhaltenen Verbindung (3) mit einer Persäure gemäß der nachfolgenden Reaktion: 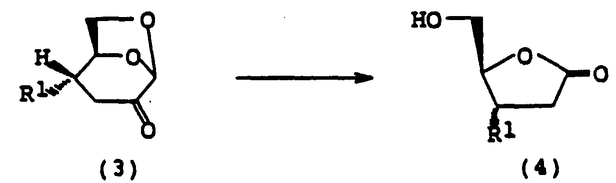 wobei R¹ die gleichen Bedeutungen, wie sie oben definiert wurden, aufweist;
(c) Erhalten der Verbindung (5) durch Umwandeln der Hydroxylgruppe der im Schritt (b) erhaltenen Verbindung (4) in eine Abspaltungsgruppe durch Veresterung, OX¹, gemäß der nachfolgenden Reaktion: 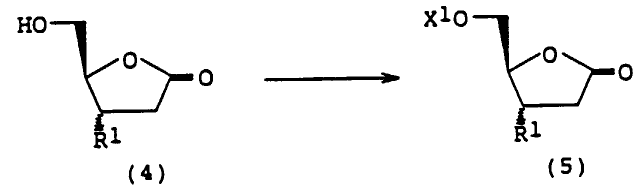 wobei R¹ die gleichen Bedeutungen wie oben definiert aufweist, und X¹ als Gruppe definiert ist, die in Form von OX¹ entfernbar ist:
(d) Erhalten der Verbindung (6) durch Behandeln der im Schritt (c) erhaltenen Verbindung (5) mit einer Base in Gegenwart von R³OH gemäß der nachfolgenden Reaktion: 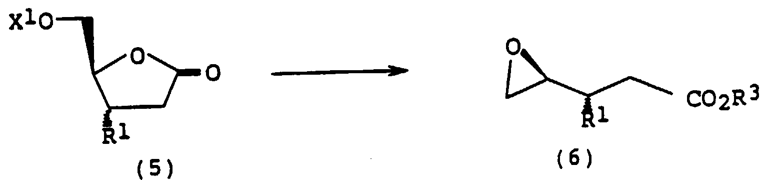 wobei R¹ und X¹ die gleichen wie oben definierten Bedeutungen aufweisen, und R³ ein Wasserstoffatom oder eine Alkylgruppe mit 1 bis 5 Kohlenstoffatomen ist;
(e) Erhalten der Verbindung (7) durch Einführen einer Alkenylgruppe in die im Schritt (d) erhaltene Verbindung (6) unter Verwendung eines Alkenylmetallsalzes gemäß der nachfolgenden Reaktion: 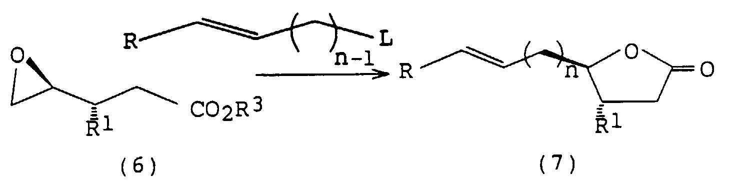 wobei R¹ und R³ die gleichen Bedeutungen wie oben definiert aufweisen, n ein Ganzzahliges von 1 bis 3 ist, R ein Wasserstoffatom oder eine Alkylgruppe mit 1 bis (8-n) Kohlenstoffatomen ist, wobei n die gleiche Bedeutung wie oben angegeben aufweist und L ein Alkalimetall oder MgX (wobei X ein Halogen ist) ist;
(f) Erhalten der Verbindung (8) durch oxidative Spaltung der Doppelbindung der Alkenylgruppe der im Schritt (e) erhaltenen Verbindung (7) gefolgt von einer Reduktion gemäß der nachfolgenden Reaktion: 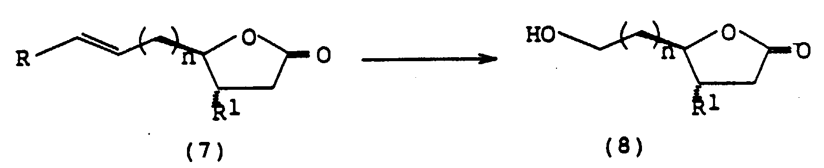 wobei R, R¹ und n die gleichen Bedeutungen wie oben angegeben aufweisen;
(g) Erhalten der Verbindung (9) durch Umwandeln der Hydroxylgruppe der im Schritt (f) erhaltenen Verbindung (8) in eine Abspaltungsgruppe, OX², gemäß der nachfolgenden Reaktion: 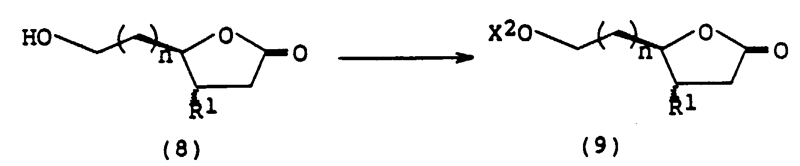 wobei R¹ und n die gleichen Bedeutungen wie oben definiert aufweisen, und X² eine Gruppe ist, die in Form von OX² entfernbar ist;
(h) Erhalten der Verbindung (10) durch eine Ringschlußreaktion der im Schritt (g) erhaltenen Verbindung (9) durch Behandeln mit einer Base gemäß der nachfolgenden Reaktion: 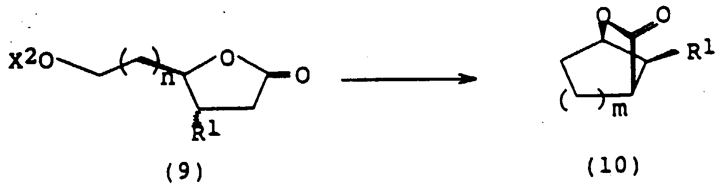 wobei R¹, m, n und X² die gleichen Bedeutungen wie oben definiert aufweisen;
(i) Erhalten der Verbindung (11) durch Reduktion der im Schritt (h) erhaltenen Verbindung (10) gemäß der nachfolgenden Reaktion: 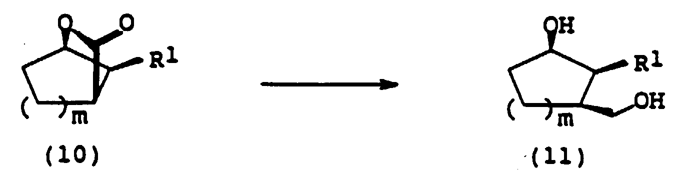 wobei R¹ und m die gleichen Bedeutungen wie oben definiert aufweisen;
(j) Erhalten der Verbindung (12) durch selektive Umwandlung der primären Hydroxylgruppe der im Schritt (i) erhaltenen Verbindung (11) in eine Abspaltungsgruppe, OX³, gemäß der nachfolgenden Reaktion: 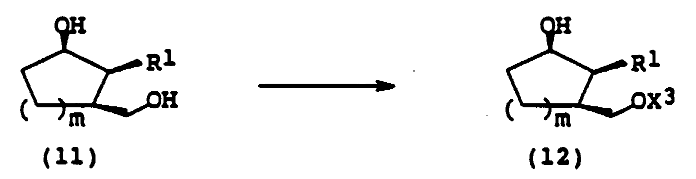 wobei R¹ und m die gleichen Bedeutungen wie oben definiert aufweisen, und X³ eine Gruppe ist, die in Form von OX³ entfernbar ist;
k) Erhalten der Verbindung (13) durch Einführen einer Schutzgruppe, R⁴, an die sekundäre Hydroxylgruppe der im Schritt (j) erhaltenen Verbindung (12) gemäß der nachfolgenden Reaktion: 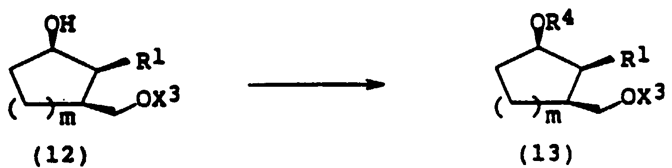 wobei R¹, m und X³ die gleichen Bedeutungen wie oben definiert aufweisen, und R⁴ eine Schutzgruppe der Hydroxylgruppe ist;
(1) Erhalten der Verbindung der chemischen Formel (14) durch Einführen einer Nitrilgruppe in die Verbindung der chemischen Formel (13) gemäß der nachfolgenden Reaktion : 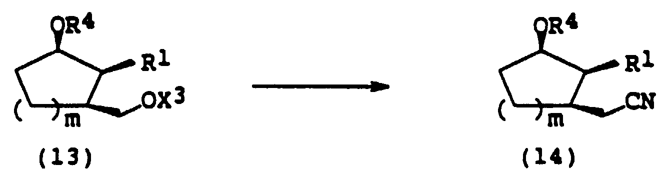 wobei R¹, R⁴, m und X³ die gleichen Bedeutungen wie oben definiert aufweisen; und
Erhalten einer gesättigten monozyklischen Kohlenwasserstoffverbindung (1) durch Hydrolyse, um die Nitrilgruppe der im Schritt (1) erhaltenen Verbindung (14) in eine Karboxylgruppe umzuwandeln, worauf sich die Entfernung der Schutzgruppe R⁴ unter sauren Bedingungen anschließt, und anschließende Veresterung der Karboxylgruppe gemäß der nachfolgenden Reaktion: 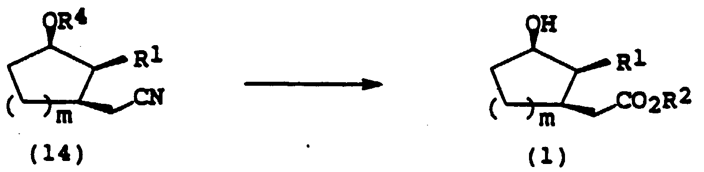 wobei R¹, R², R⁴ und m die gleichen Bedeutungen wie oben definiert aufweisen.

2. Verfahren zur Herstellung einer gesättigen monozyklischen Kohlenwasserstoffverbindung mit den Schritten (a) bis (m) gemäß Anspruch 1, dadurch gekennzeichnet, daß die Verbindung (1) durch die nachfolgende allgemeine chemische Formel (1') dargestellt wird: 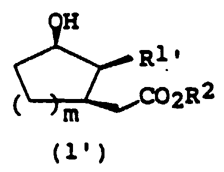 wobei R² und m die gleichen Bedeutungen wie oben angegeben aufweisen, und R^{1'} eine Alkenylgruppe mit 2 bis 10 Kohlenstoffatomen ist, und wobei eine Alkenylgruppe an die Enonstelle an die Stelle der im Schritt (a) verwendeten Alkylgruppe eingefügt wird; das Produkt wie im Schritt (b) oxidiert wird, wodurch eine Epoxidation der Doppelbindung und die Bildung eines Lactonrings stattfindet, worauf das Produkt weiter oxidiert wird, um die Kohlenstoff-Kohlenstoff-Bindung des Epoxyrings oxidativ zu spalten, und anschließend ein Schutz der Hydroxylguppe und eine Acetalisierung durchgeführt werden; und im Schritt (m) eine Wittig-Reaktion durchgeführt wird, nachdem eine Hydrolyse der Nitrilgruppe, eine Abspaltung der Schutzgruppe und eine Veresterung durchgeführt wurden.

3. Verfahren nach Anspruch 1, dadurch gekennzeichnet, daß der Schritt (a) unter Verwendung eines Metallalkylats in Gegenwart eines Kupfer(I)-Salzes durchgeführt wird.

4. Verfahren nach Anspruch 2, dadurch gekennzeichnet, daß der Schritt (a) unter Verwendung eines Metallalkenylats in Gegenwart eines Kupfer(I)-Salzes durchgeführt wird.

5. Verfahren nach Anspruch 1 oder Anspruch 2, dadurch gekennzeichnet, daß der Schritt (e) unter Verwendung eines Metallalkenylats in Gegenwart eines Kuper(I)-Salzes durchgeführt wird.

6. Verfahren nach Anspruch 3, dadurch gekennzeichnet, daß das Metallalkylat ein Alkyllithium oder ein Alkylmagnesiumhalogenid mit 1 bis 10 Kohlenstoffatomen ist.

7. Verfahren nach Anspruch 4, dadurch gekennzeichnet, daß das Metallalkenylat ein Alkenyllithium oder ein Alkenylsmagnesiumhalogenid mit 2 bis 10 Kohlenstoffatomen ist.

8. Verfahren nach Anspruch 5, dadurch gekennzeichnet, daß das Metallalkenylat ein Alkenyllithium oder ein Alkenylmagnesiumhalogenid mit 2 bis 10 Kohlenstoffatomen ist.

9. Verfahren nach Anspruch 3 oder Anspruch 4, dadurch gekennzeichnet, daß das Kupfer(I)-Salz Kupfer(I)-iodid, Kupfer(I)-bromid und Kupfer(I)-chlorid ist.

10. Verfahren nach Anspruch 1 oder Anspruch 2, dadurch gekennzeichnet, daß die Gruppen X¹, X² und X³ in den Schritten (c), (g) und (j) durch eine Veresterungsreaktion eingeführt werden.

11. Verfahren nach Anspruch 1, dadurch gekennzeichnet, daß die Verbindung (1) durch die nachfolgende chemische Formel dargestellt wird:

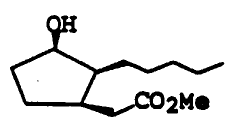

12. Verbindung der nachfolgenden allgemeinen Formel (10): 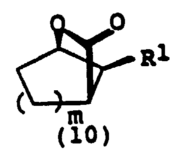 wobei R¹ eine Alkylgruppe mit 1 bis 10 Kohlenstoffatomen oder eine Alkenylgruppe mit 2 bis 10 Kohlenstoffatomen ist und m eine Ganzzahliges von 1 bis 3 ist.

## Revendications

1. Procédé de préparation d'un composé hydrocarboné monocyclique saturé représenté par la formule chimique (1) suivante : 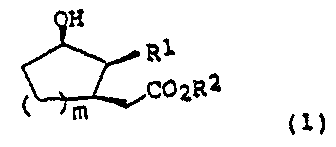 dans laquelle R¹ représente un groupe alkyle contenant de 1 à 10 atomes de carbone ou un groupe alcényle contenant de 2 à 10 atomes de carbone, R² représente un groupe alkyle contenant de 1 à 5 atomes de carbone, et m est un nombre entier compris entre 1 et 3,
ledit procédé comprenant les étapes consistant à :
(a) obtenir le composé (3) en ajoutant un groupe alkyle ou un groupe alcényle à un site énone de la lévoglucosénone (2), comme le montre la réaction suivante : 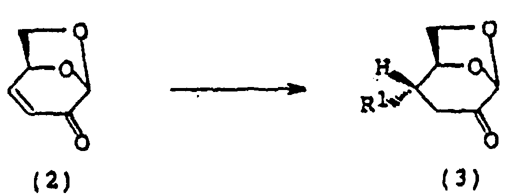 dans laquelle R¹ est tel que défini ci-dessus ;
(b) obtenir le composé (4) en oxydant le composé (3) obtenu dans l'étape (a) avec un peracide, comme le montre la réaction suivante : 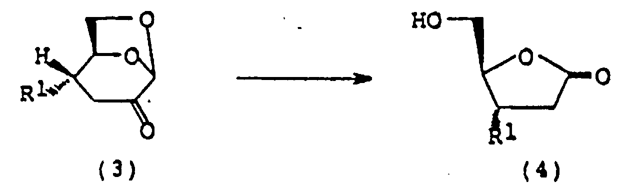 dans laquelle R¹ est tel que défini ci-dessus ;
(c) obtenir un composé (5) en convertissant le groupe hydroxyle du composé (4) obtenu dans l'étape (b) en un groupe partant par estérification, OX¹, comme le montre la réaction suivante : 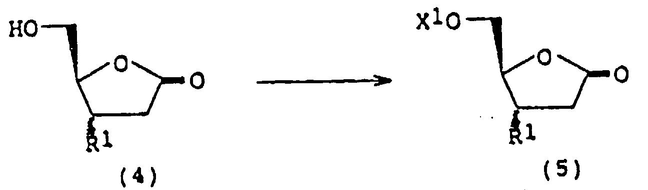 dans laquelle R¹ est tel que défini ci-dessus, et X¹ est défini comme un groupe capable d'être retiré sous la forme OX¹ ;
(d) obtenir un composé (6) en traitant le composé (5) obtenu dans l'étape (c) par une base en présence de R³OH, comme le montre la réaction suivante : 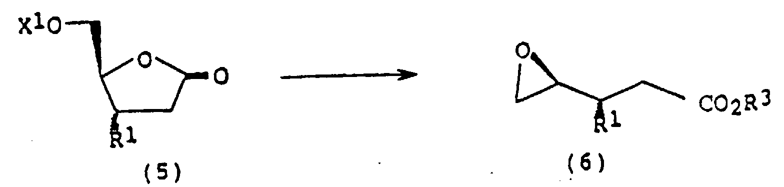 dans laquelle
R¹ et X¹ sont tels que définis ci-dessus, et R³ représente un atome d'hydrogène ou un groupe alkyle contenant de 1 à 5 atomes de carbone ;
(e) obtenir un composé (7) en introduisant un groupe alcényle dans le composé (6) obtenu dans l'étape (d) en utilisant un sel d'alcénylmétal, comme le montre la réaction suivante : 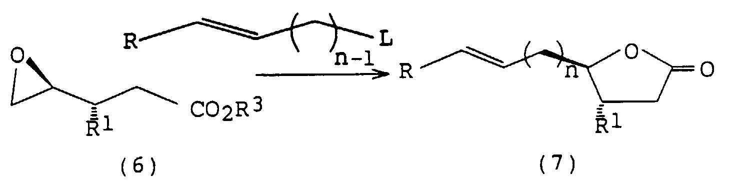 dans laquelle R¹ et R³ sont tels que définis ci-dessus, n est un nombre entier valant de 1 à 3, R représente un atome d'hydrogène ou un groupe alkyle contenant de 1 à (8-n) atomes de carbone, dans lequel n est tel que défini ci-dessus et L est un métal alcalin ou MgX (dans lequel X représente un atome d'halogène)
(f) obtenir un composé (8) par coupure oxydative de la double liaison du groupe alcényle du composé (7) obtenu dans l'étape (e), puis réduire, comme le montre la réaction suivante. 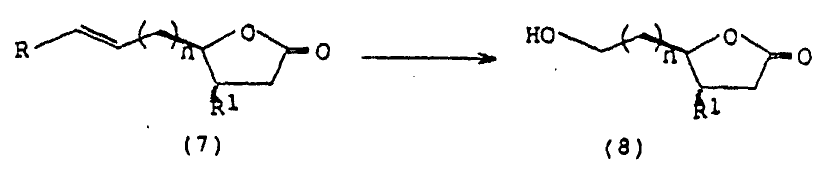 dans laquelle R, R¹ et n sont tels que définis ci-dessus ;
(g) obtenir un composé (9) en convertissant le groupe hydroxyle du composé (8) obtenu dans l'étape (f) en un groupe partant, OX², comme le montre la réaction suivante : 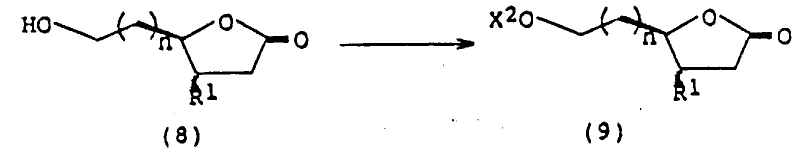 dans laquelle R¹ et n sont tels que définis ci-dessus, et X² est un groupe pouvant être retiré sous la forme de OX² ;
(h) obtenir un composé (10) en effectuant une réaction de fermeture de cycle du composé (9) obtenu dans l'étape (g) par traitement par une base comme le montre la réaction suivante : 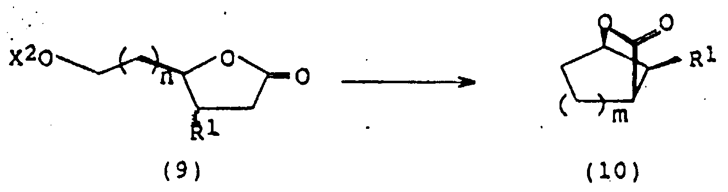 dans laquelle R¹, m, n, X² sont tels que définis ci-dessus ;
(i) obtenir un composé (11) en réduisant le composé (10) obtenu dans l'étape (h) comme le montre la réaction suivante : 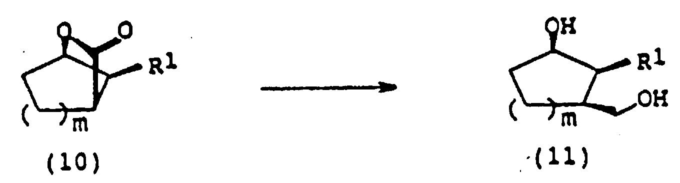 dans laquelle R¹ et m sont tels que définis ci-dessus ;
(j) obtenir un composé (12) en convertissant sélectivement le groupe hydroxyle primaire du composé (11) obtenu dans l'étape (i) en un groupe de partant, OX³, comme le montre la réaction suivante : 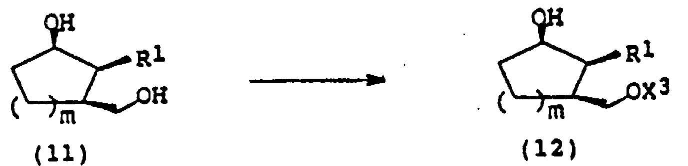 dans laquelle R¹ et m sont tels que définis ci-dessus, et X³ est un groupe pouvant être retiré sous la forme de OX³ ;
(k) obtenir un composé (13) en introduisant un groupe protecteur, R⁴, sur le groupe hydroxyle secondaire du composé (12) obtenu dans l'étape (j) comme le montre la réaction suivante : 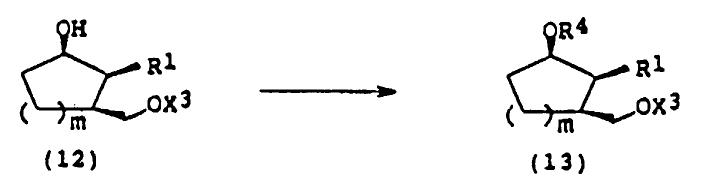 dans laquelle, R¹, m et X³ sont tels que définis ci-dessus et R⁴ est un groupe protecteur de groupe hydroxyle ;
(1) obtenir un composé représenté par la formule chimique (14) en introduisant un groupe nitrile dans le composé représenté par la formule chimique (13) comme le montre la réaction suivante : 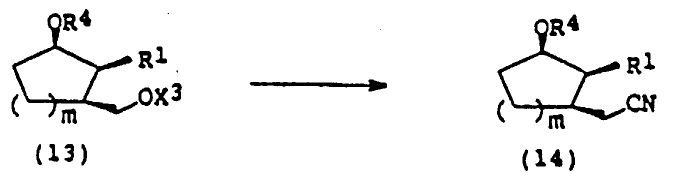 dans laquelle R¹, R⁴, m, et X³ sont tels que définis ci-dessus ; et
(m) obtenir un composé hydrocarboné monocyclique saturé (1) par hydrolyse pour convertir le groupe nitrile du composé (14) obtenu dans l'étape (1) en un groupe carboxyle, puis en retirant le groupe protecteur R⁴, en condition acide, et en effectuant ensuite l'estérification du groupe carboxyle, comme le montre la réaction suivante : 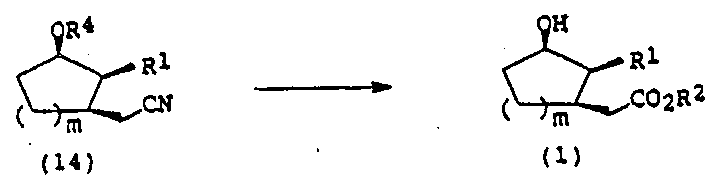 dans laquelle R¹, R², R⁴, et m sont tels que définis ci-dessus.

2. Procédé de préparation d'un composé hydrocarboné monocyclique saturé comprenant lesdites étapes (a) à (m) conformément à la revendication 1, caractérisé en ce que ledit composé (1) est représenté par la formule chimique générale (1') suivante : 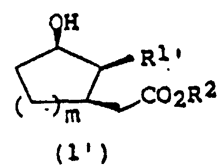 dans laquelle R² et m sont tels que définis ci-dessus, et R^{1'} est un groupe alcényle contenant de 2 à 10 atomes de carbone, et dans lequel,
on ajoute un groupe alcényle au site énone à la place du groupe alkyle utilisé dans l'étape (a) ; on oxyde le produit comme dans l'étape (b) ce qui entraîne l'époxydation de la double liaison et la formation d'un cycle lactone, puis on oxyde ensuite ledit produit afin de couper la liaison carbone-carbone du cycle époxy par oxydation, puis on effectue une protection du groupe époxyle et une acétalation ; et on effectue une réaction de Wittig dans l'étape (m) après une hydrolyse du groupe nitrile, une déprotection et une estérification.

3. Procédé conforme à la revendication 1, caractérisé en ce que l'étape (a) est effectuée en utilisant un alkylate de métal en présence d'un sel de cuivre (I).

4. Procédé conforme à la revendication 2, caractérisé en ce que l'étape (a) est effectuée en utilisant un alcénylate de métal en présence d'un sel de cuivre (I).

5. Procédé conforme à l'une quelconque des revendications 1 ou 2, caractérisé en ce que ladite étape (e) est effectuée en utilisant un alcénylate de métal en présence d'un sel de cuivre (I).

6. Procédé conforme à la revendication 3, caractérisé en ce que ledit alkylate de métal est un halogénure d'alkyllithium ou d'alkylmagnésium contenant de 1 à 10 atomes de carbone.

7. Procédé conforme à la revendication 4, caractérisé en ce que ledit alcénylate de métal est un halogénure d'alcényllithium ou d'alcénylmagnésium contenant de 2 à 10 atomes de carbone.

8. Procédé conforme à la revendication 5, caractérisé en ce que ledit alcénylate de métal est un halogénure d'alcényllithium ou d'alcénylmagnésium contenant de 2 à 10 atomes de carbone.

9. Procédé conforme à l'une quelconque des revendications 3 ou 4, caractérisé en ce que le sel de cuivre (I) est de l'iodure de cuivre (I), du bromure de cuivre (I), ou du chlorure de cuivre (I).

10. Procédé conforme à l'une quelconque des revendications 1 et 2, caractérisé en ce que les groupes X¹, X², et X³ dans lesdites étapes (c), (g) et (j) sont introduits par une réaction d'estérification.

11. Procédé conforme à la revendication 1, caractérisé en ce que ledit composé (1) est représenté par la formule chimique suivante :

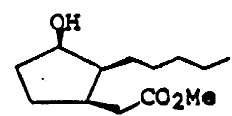

12. Composé représenté par la formule chimique générale (10) suivante : 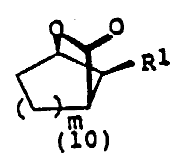 dans laquelle R¹ représente un groupe alkyle contenant de 1 à 10 atomes de carbone ou un groupe alcényle contenant de 2 à 10 atomes de carbone et m est un nombre entier compris entre 1 et 3.
